# EUROPÄISCHE PATENTSCHRIFT

(11) **EP 0 698 602 B1**
(45) Veröffentlichungstag und Bekanntmachung des Hinweises auf die Patenterteilung: **06.02.2002**
(21) Anmeldenummer: 95117215.4
(22) Anmeldetag: 04.03.1991
(51) Int. Cl.: C07D 223/22

(54) **Verfahren zur Herstellung von 5-Chlorcarbonyl-5H-dibenz/b,f/azepin**
Process for the preparation of 5-chlorocarbonyl-5H-dibenz(b,f)azepine
Procédé pour la préparation de 5-chlorocarbonyl-5H-dibenz(b,f)azépine

(30) Priorität: 05.07.1990 DE 90342510
(43) Veröffentlichungstag der Anmeldung: 28.02.1996
(62) Teilanmeldung aus: 91103198.7
(73) Patentinhaber: AWD.pharma GmbH & Co.KG, 01097 Dresden (DE)
(72) Erfinder: Palitzsch, Peter, Dipl.Chem., D-01129 Dresden (DE); Müller, Rainer, Dr., D-01309 Dresden (DE); Richter, Ehrhard, Dr., D-01445 Radebeul (DE)
(74) Vertreter: Grabherr, Claudia, Dipl.Ing.

(56) Entgegenhaltungen:
- EP-A- 0 466 972
- DE-B- 1 136 707
- CHEMICAL ABSTRACTS, vol. 113, no. 1, 2.Juli 1990 Columbus, Ohio, US; abstract no. 6185n, Seite 600; & PL-A-147 078 (STAROGARDZKIE ZAKLADY FARMACEUTYCZNE POLFA) 29.April 1989
- CHEMICAL ABSTRACTS, vol. 117, no. 15, 12.Oktober 1992 Columbus, Ohio, US; abstract no. 150906y, Seite 818; & DD-A-298 508 (ARZNEIMITTELWERK DRESDEN GMBH)
- CHEMICAL ABSTRACTS, vol. 117, no. 17, 26.Oktober 1992 Columbus, Ohio, US; abstract no. 171249b, Seite 824; & DD-A-300 169 (ARTNEIMITTELWERK DRESDEN GMBH)

## Beschreibung

Die Erfindung betrifft ein im großtechnischen Maßstab leicht durchführbares Verfahren zur Herstellung von 5-Chlorcarbonyl-5H-dibenz/b,f/azepin, einer Vorstufe für die Synthese von 5-Carbamoyl- 5H-dibenz/b,f/azepin. 5-Carbamoyl-5H-dibenz/b,f/azepin besitzt eine hohe Bedeutung als Arzneimittel , das vorzugsweise als Antiepileptikum, verwendet wird.

Die Synthese des 5-Carbamoyl-5H-dibenz/b,f/azepins (III) aus Iminostilben (I) (= 5H-Dibenz/b,f/azepin) wurde erstmalig von W. Schindler (DE-AS 1.136.707, CH-PS 54.023) beschrieben.

Nach diesem Verfahren wird das Iminostilben (I) in Toluen suspendiert, und in diese Suspension wird Phosgen eingeleitet, wobei sich die Reaktionsmischung bis auf 70 °C erwärmt.

Anschließend wird die Reaktionsmischung unter weiterem Einleiten von Phosgen zum Rückfluß angeheizt und so lange gekocht, bis das Iminostilben vollständig umgesetzt und die Chlorwasserstoffentwicklung beendet ist.

Sobald die Reaktionslösung frei von Iminostilben ist, wird die Phosgeneinleitung abgestellt. Das überschüssige Phosgen wird aus der Reaktionsmischung mit trockenem Stickstoff oder trockener Luft entfernt. (S. hierzu DE-AS 1.001.271, wonach das überschüssige Phosgen nach der beendeten Phosgenierung des 5H-10,11-Dihydrodibenz/b,f/azepins = Iminodibenzyl gleichfalls mit trockener Luft ausgeblasen wird). Die derartig entgiftete Reaktionslösung wird wie üblich aufgearbeitet und das isolierte 5-Chlorcarbonyl-5H-dibenz/b,f/azepin (II) in bekannter Weise zum 5-Carbamoyl-5H-dibenz/b,f/ azepin (III) amidiert.

Die bis zum heutigen Zeitpunkt beschriebenen Verfahren arbeiten alle in inerten wasserfreien Lösungsmitteln bei Temperaturen über 100 °C (beispielsweise in Toluen, in Chlorbenzen oder in o-Dichlorbenzen; siehe hierzu in Betracht gezogene Patentschriften und Übersichtsartikel von B. RENFROE, C. HARRINGTON und G. R. PROCTOR in "Heterocyclic compounds", Vol. 43, "Azepines", Teil I, John Wiley & Sons, New York 1984, Seite 524, Tabelle 118).

Die Dissoziation des bei der Phosgenierung stets entstehenden Iminostilben-Hydrochlorids in Chlorwasserstoffgas und freies Iminostilben erfolgt bei allen technischen Verfahren thermisch, indem man das Reaktionsgemisch bis zum Siedepunkt des indifferenten Lösungsmittels anheizt und unter Rückfluß Phosgen einleitet.

Die nach dem Stand der Technik durchgeführten Hochtemperatur-Phosgenierungen arbeiten alle bei 100 °C und höher, um eine vollständige Phosgenierung des Iminostilbens oder Iminodibenzyls zu erreichen.

Die bekannten Verfahren zur Herstellung von Carbamidsäurechloriden aus sekundären Aminen sind in HOUBEN-WEYL (Band E 4, Seite 46 - 50, 1983) in einer Tabelle zusammengestellt. Als Lösungsmittel werden in der Regel vor allem aromatische Kohlenwasserstoffe wie Benzen, Toluen oder Chlorbenzen eingesetzt.

Arbeitet man bei niederen Temperaturen, so wird bei der Einleitung von Phosgen in eine Lösung des sekundären Amins in einem inerten Lösungsmittel nur die Hälfte der Aminmenge in das gewünschte Carbamidsäurechlorid überführt, weil der bei der Reaktion freiwerdende Chlorwasserstoff ein Hydrochlorid bildet. Das Amin-Hydrochlorid scheidet sich in kristalliner Form ab, d. h. die Ausbeute an Carbamidsäurechlorid kann im günstigsten Fall nur 50 % betragen.

Seit der Arbeit von H. ERDMANN und P.HUTH [J. Prakt. Chem. (2) 56, 7(1897)] ist bekannt, daß man den Umsatz vervollständigen kann, wenn man eine inerte wasserfreie Base wie Pyridin in mindestens äquimolekularer Menge zusetzt. Nach HOUBEN-WEYL (s. o.) sind als inerte Basen außer Pyridin noch Triethylamin und naturgemäß das umzusetzende Amin selbst geeignet. Da man stets mindestens äquimolekulare Mengen an inerter Base benötigt, verteuert sich die Kaltphosgenierung und der Verfahrensablauf wird technologisch aufwendig, denn das Amin-Hydrochlorid muß auf jeden Fall zwecks Rückgewinnung der inerten Base abgetrennt werden.

Aus diesem Grund besitzt die Kaltphosgenierung in Gegenwart von inerten Hilfsbasen nur Bedeutung für die Umsetzung von temperaturempfindlichen sekundären Aminen, die bei den hohen Temperaturen der Heißphosgenierung verstärkt zu unerwünschten Nebenreaktionen neigen.

In der Technik arbeitet man bevorzugt bei Temperaturen oberhalb von 100 °C. Im HOUBEN-WEYL (s. o.) wird hierzu ausgeführt:
"Vorteilhafterweise erhitzt man das Reaktionsgemisch unter weiterem Einleiten von Phosgen auf über 100 °C, wobei das Amin-Hydrochlorid in das Carbamidsäurechlorid übergeht."

Das bei dieser thermischen Dissoziation freiwerdende HCl-Gas reißt beträchtliche Mengen des extrem giftigen Phosgens mit. Aus diesem Grund muß das Abgas in speziellen Abgasanlagen entgiftet bzw. vernichtet werden. Im Haveriefall kann eine derartige Verfahrensweise zu einer ernsthaften Gefährdung der Umwelt führen.

Die Arbeitsweise der Heißphosgenierung hat vor allem die folgenden schwerwiegenden Nachteile:
- hohe Abgasbelastungen durch freigesetztes HCl-Gas, durch mitgerissenes Phosgen und mitgeführte Lösungsmitteldämpfe und die daraus resultierenden Umweltschutzprobleme
- lange Reaktionszeiten über 18 - 24 Stunden mit starken korrosiven Belastungen der Anlagen
- hohe energetische Aufwendungen und
- eine Vielzahl von Nebenreaktionen sowie Dunkelfärbung des Reaktionsproduktes, die letzten Endes zu entscheidenden Qualitätsminderungen des 5-Carbamoyl-5H-dibenz/b,f/ azepins führen sowie die
- oberhalb von 90 °C verstärkt stattfindende Verengung des Siebenringes des Iminostilbens unter Bildung von unerwünschtem 9-Methylacridin.

Das Iminostilben ist in die Gruppe der temperaturempfindlichen Amine einzuordnen. Aus diesen Gründen wird Iminostilben vorteilhafferweise nach dem Verfahren von SCHINDLER (DE-AS 1.136.707) phosgeniert.

Die thermische Dissoziation des Iminostilben-Hydrochlorids in freies Iminostilben und HCl-Gas erfolgt erst bei ca. 90 °C hinreichend schnell, um für industrielle Zwecke akzeptable Reaktionszeiten zu erreichen.

Die von SCHINDLER bevorzugte Variante der Zweiteilung der Phosgenierung in eine Phase der Kaltphosgenierung (Umsatz der ersten Hälfte, 50 %) und eine Phase der Heißphosgenierung (Umsatz der zweiten Hälfte) besitzt gegenüber der direkten Heißphosgenierung eindeutige Vorteile, denn erstens wurde auf diese Weise die Ausbeute beträchtlich erhöht, zweitens wurden die oberhalb von 90 °C ablaufenden Nebenreaktionen zurückgedrängt und drittens wurden die Qualität und die Farbe verbessert. Trotzdem bleiben aber die beschriebenen Nachteile in der zweiten Reaktionsstufe - von Beginn des Anheizens auf 90 °C und während der thermischen Dissoziation des Iminostilben-Hydrochlorids bis zum Endpunkt der Reaktion - erhalten.

Um die schonenden Reaktionsbedingungen der Kaltphosgenierungsphase möglichst vollständig auszunutzen, wird ein Phosgenüberschuß in die Reaktionsmischung eingeleitet. Heizt man anschließend die Reaktionsmischung zur thermischen Dissoziation des lminostilben-Hydrochlorids an, so kann es zu einem Druckstoß kommen.

Auf diese Gefahr wird im HOUBEN-WEYL (Band E4, Seite 744, 1983) ebenfalls aufmerksam gemacht.

Bei einem Druckstoß reißen die spontan freigesetzten HCl-Gas-mengen beträchtliche Mengen Phosgen mit. Die Abgas-Vemichtungsanlage bzw. -Entgiftungsanlage muß folglich groß genug dimensioniert sein, um einen Austritt des Phosgens in die Atmosphäre zu verhindem.

Um die unerwünschten Nebenreaktionen und die Bildung von 9-Methylacridin zurückzudrängen, arbeitet man zweckmäßig bei Temperaturen von 90 - 100 °C. Bei dieser Reaktionsführung verläuft zwar die Phosgenierung hinreichend schnell, aber der Dampfdruck des Phosgens ist gegenüber der Phase der Kaltphosgenierung naturgemäß beträchtlich erhöht, so daß es nicht zu verhindern ist, daß der freigesetzte Chlorwasserstoff ständig große Mengen Phosgen mitführt. Dies äußert sich schon in der Tatsache, daß für den Umsatz der ersten Hälfte Iminostilben in der Phase der Kaltphosgenierung wesentlich weniger Zeit benötigt wird als für den Umsatz der zweiten Hälfte in der Phase der Heißphosgenierung.

Nach dem vollständigem Umsatz des Iminostilbens muß die Reaktionslösung entgiftet werden. In der Regel wird das überschüssige Phosgen aus der heißen Reaktionslösung mit trockenem Stickstoff ausgeblasen, oder es wird ein Teil des Lösungsmittels abdestilliert, bis die Reaktionsmischung phosgenfrei ist. Diese Verfahrensweisen der Entgiftung besitzen den Nachteil, daß bei Undichtigkeiten aufgrund des in der Anlage herrschenden Gasdruckes Phosgen austreten kann. Über längere Zeiträume besteht folglich die Gefahr, daß die Umwelt durch Phosgen belastet werden kann.

In der Literatur sind außer dem beschriebenen Verfahren zur direkten Phosgenierung des Iminostilbens (I) noch einige Verfahren zur Synthese des 5-Carbamoyl-5H-dibenz/b,f/azepins (III) beschrieben worden, die vom Iminodibenzyl (IV) (= 10,11-Dihydro-5H-dibenz/b,f/azepin) ausgehen (siehe hierzu Patentschriften GB 1.246.606, DD 82.719, DD 100.948, DD 101.671, DD 102.149, DD 102.150, DD 102.151, DD 108.535, DD 133.052, DD 234.862 A1, DD 234.863 A1). Nach diesen Verfahren wird Iminodibenzyl (IV) in einem siedenden aromatischen Lösungsmittel - vorzugsweise Toluen oder Chlorbenzen - mit Phosgen umgesetzt, wobei das Phosgen in den Rücklauf eingeleitet wird. Es handelt sich folglich wieder um eine Heißphosgenierung mit allen ihren Nachteilen.

Das erhaltene 5-Chlorcarbonyl-5H-10,11-dihydro-dibenz/b,f/azepin (V) wird anschließend in einem inerten organischen Lösungsmittel mit elementarem Brom oder einem selektiven Bromierungsreagenz umgesetzt, wobei die entsprechenden 10-Monobrom - (VI, R=H) und/oder 10,11-Dibrom-Derivate (VII, R=Br) gebildet werden. Anschließend werden die Brom-Verbindungen (VI und/oder VII) thermisch dehydrobromiert und/oder debromiert. Bei diesem thermischen Prozeß erfolgt gleichzeitig ein teilweiser Austausch (30 - 40 %) des Chloratoms der 5-Chlorcarbonyl-Gruppe gegen ein Brom-Atom.

Diese drastischen Reaktionsbedingungen führen zwangsläufig aufgrund der erforderlichen hohen Reaktionstemperaturen (150 - 170 °C) und des freiwerdenden Broms zu nicht kontrollierbaren Nebenreaktionen (Kembromierungen, Verharzungen, Verfärbung, Crackung).

Daraus folgt, daß das über derartige Prozesse synthetisierte 5-Chlorcarbonyl-5H-dibenz/b,f/azepin (II) außer zahlreichen - vor allem bromhaltigen - Nebenprodukten noch schmierige teerige Anteile und Farbstoffe enthält, deren Entfernung einen enormen Aufwand erfordert bzw. bei durchgängigem Arbeiten auf 5-Carbamoyl-5H-dibenz/b,f/azepin (III) zwangsläufig zu Qualitätseinbußen beim Finalprodukt führt.

Über die Natur und Struktur der erwähnten Nebenprodukte liegen keine Kenntnisse vor. Die üblichen Reinigungsverfahren führen zu beträchtlichen Verlusten.

Bisher existiert kein Reinigungsverfahren, daß in ökonomisch vertretbarer Weise das Problem des Restbromgehaltes löst. Das alles ist erklärbar, denn der laut HPLC ermittelte Gehalt des auf diese Weise synthetisierten 5-Chlorcarbonyl-5H-dibenz/b,f/ azepins (II) liegt durchschnittlich bei 90 %, d. h. das Vorprodukt - für das Finalprodukt Carbamazepin - enthält nach dem beschriebenen Verfahren 10 % Verunreinigungen.

Das nach dem oben beschriebenen Verfahren hergestellte 5-Chlor-carbonyl-5H-dibenz/b,f/azepin wird als Substanz isoliert, indem man entweder die heiße Lösung mit Aktivkohle behandelt, filtriert und das Produkt nach der Kristallisation isoliert oder indem man nach der Filtration das Lösungsmittel abdestilliert, die Schmelze des Produktes in ein Fällbad (in ein anderes geeignetes Lösungsmittel) einlaufen läßt und anschließend das kristallisierte Produkt abtrennt.

Die Erfindung verfolgt die Aufgabe, 5-Chlorcarbonyl-5H-dibenz/ b,f/azepin (II) direkt aus Iminostilben in quantitativer Ausbeute und verbesserter Qualität herzustellen, wobei das neuartige Verfahren mit wesentlich verkürzter Reaktionszeit unter Vermeidung der Heißphosgenierungsphase bzw. unter Ausschaltung der bisher üblichen thermischen Dissoziation des Iminostilbenhydrochlorids ablaufen soll. Mit der Erfindung sollen durch die Reaktionsführung femer die Phosgenemission beseitigt und eine Selbstentfgiftung der Reaktionslösung gewährleistet werden, womit eine deutliche Verbesserung des Umweltschutzes und merklich verringerte Gefährdungsmomente erzielt werden.

Gemäß dem erfindungsgemäßen Verfahren wird in eine Suspension von Iminostilben (I) in einem inerten aromatischen Lösungsmittel bei 20 - 60 °C Phosgen eingeleitet, oder man läßt eine Lösung von Phosgen im gleichen Lösungsmittel zulaufen, bis das Iminostilben zu einem nahezu äquimolekularen Gemisch aus 5-Chlorcarbonyl-5H-dibenz/b,f/azepin (II) und Iminostilben-Hydrochlorid umgesetzt ist, worauf man durch Zugabe einer wäßrigen Base aus dem gebildeten Iminostilben-Hydrochlorid Iminostilben freisetzt und so bei Aufrechterhaltung eines sauren Reaktionsmilieus der vollständigen Phosgenierung zugänglich macht.

Vorzugsweise wird bei der Phosgenierung im Temperaturbereich von 35 - 60 °C gearbeitet. Nach dem etwa 50 %-igem Umsatz des Iminostilbens zu dem gewünschten Carbamidsäurechlorid (II) beginnt man unter ständigem weiterem Einleiten von Phosgen entweder mit dem Zulauf einer verdünnten Lösung eines Alkalihydroxides oder von verdünntem Ammoniakwasser.

Während des Zulaufes der wäßrigen Base wird die Temperatur bei 35-60 °C gehalten. Nach dem Zulauf der wäßrigen Base wird die Einleitung des Phosgens bis zum 100 %-igen Umsatz des Iminostilbens fortgesetzt, wobei die angegebenen Temperaturgrenzen eingehalten werden. Der vollständige Umsatz des Iminostilbens wird dünnschichtchromatographisch ermittelt.
In der Endphase der Phosgenierung hält man die Temperatur bei etwa 40 - 50 °C (in Chlorbenzen) bzw. bei 50 - 60 °C (in Toluen, wobei leicht höhere Temperaturen mitunter angewandt werden müssen, um das gebildete 5-Chlorcarbonyl-5H-dibenz/b,f/azepin in Lösung zu halten), damit das gebildete Carbamidsäurechlorid (II) nicht auskristallisiert. Temperaturabweichungen um einige Grad nach oben oder unten besitzen keine Bedeutung.

Sobald in der Reaktionslösung kein Iminostilben mehr nachweisbar ist, wird die Phosgeneinleitung beendet. Danach wird die stark saure Reaktionsmischung ( pH-Wert = 1) langsam auf 80 - 90 °C erwärmt, so daß das überschüssige Phosgen durch die in der wäßrigen Phase vorhandene Salzsäure hydrolysiert wird. Auf diese Weise wird die Reaktionsmischung in wesentlich kürzerer Zeit und ungefährlicher entgiftet als nach dem Ausblasverfahren der unter Ausschluß von Wasser durchgeführten Heißphosgenierung.

Weder das bereits gebildete 5-Chlorcarbonyl-5H-dibenz/b,f/azepin (II) noch das eingeleitete Phosgen werden durch die zugesetzte wäßrige Base oder die gegen Ende der Phosgenierung vorliegende stark salzsaure wäßrige Phase in irgendeiner Form merklich zersetzt. Phosgen wird beispielsweise mit 15 - 20 %-iger Natronlauge entgiftet (s. "Organikum", 6. Auflage, S. 630, 1967).

Das 5-Chlorcarbonyl-5H-dibenz/b,f/azepin (II) wird als alkaliempfindliches Carbamidsäurechlorid ebenfalls in Gegenwart von verdünnten wäßrigen Alkalilaugen sofort spontan über das Natriumsalz der betreffenden Carbamidsäure unter Decarboxylierung wieder in Iminostilben überführt.
Das sind die Gründe, warum man Amine normalerweise nicht in Gegenwart wäßrig-alkalischer Lösungen mit Phosgen umsetzen kann. In völlig unerwarteter Weise verläuft die Phosgenierung des Iminostilbens unter den beschriebenen Bedingungen außerdem wesentlich schneller und unter deutlich milderen, schonenderen Bedingungen. Die Reaktionszeit wird nach dem beschriebenen Weg auf die Hälfte verkürzt. Die Bildung der Nebenprodukte wird ebenfalls stark zurückgedrängt. Unter den beschriebenen Reaktionsbedingungen wird erstaunlicherweise nur sehr wenig 9-Methylacridin gebildet. Dieses unerwünschte Nebenprodukt wird nach dem beschriebenen Verfahren auf sehr elegante Art vorteilhafterweise nach der "Selbstentgiftung" bei der Phasentrennung entfernt.
Die entgiftete heiße Reaktionslösung bleibt zur Phasentrennung bei 80 - 90 °C eine gewisse Zeit stehen. Die untere wäßrige stark saure Phase wird abgetrennt und verworfen. In dieser wäßrigen Phase sind - außer dem jeweiligen Salz - ungefähr 0,2 % der ursprünglich eingesetzten Iminostilbenmenge in Form der verschiedensten Nebenprodukte enthalten. Davon entfallen schätzungsweise 50 % auf 9-Methylacridin, das als Hydrochlorid vorliegt.

Die Entgiftung der Reaktionslösung durch die gebildete 7 - 10 %-ige Salzsäure (die jedoch bei übermäßiger Einleitung von Phosgen sogar maximal konzentriert sein kann) besitzt folglich noch einen positiven Effekt, indem basische aminartige Nebenprodukte in Form ihrer Hydrochloride aus der organischen Phase extrahiert werden, d. h. die wäßrig-saure Phase wirkt in bezug auf das Endprodukt 5-Carbamoyl-5H-dibenz/b,f/azepin (III) im Sinne einer Vorreinigung.
Als inerte Lösungsmittel eignen sich sowohl die aromatischen Kohlenwasserstoffe Benzen, Toluen oder Chlorbenzen als auch die aliphatischen Chlorkohlenwasserstoffe Chloroform oder Tetrachlorkohlenstoff, wobei vorzugsweise Toluen oder Chlorbenzen verwendet werden.
Als Basen werden vorzugsweise verdünnte Alkalilaugen oder verdünntes Ammoniakwasser eingesetzt. Die Verwendung dieser Basen -als Vorzugsvariante- garantiert ein abgasfreies Phosgenierungsverfahren bis zum nahezu vollständigen Umsatz des Iminostilbens.
Auch der Einsatz von wäßrigen Lösungen der Alkalikarbonate bzw. -hydrogenkarbonate ist prinzipiell möglich. In diesem speziellem Fall setzt aber mit dem Beginn des Zulaufs sofort eine Kohlendioxidentwicklung ein , so daß spätestens nach dem 50%igen Umsatz Phosgen in das Abgas gelangt. Obwohl hier weniger Phosgen ausgetragen wird als nach dem Heißphosgenierungsverfahren, stellt die Verwendung von Alkalilaugen oder Ammoniakwasser die bevorzugte Verfahrensvariante dar.
Der Einsatz der letztgenannten Basen ist jedoch von Reaktionsbeginn an nicht möglich , da unter alkalischen Bedingungen - d.h. in Gegenwart überschüssiger wäßriger Baselösungen - das Phosgen sofort zersetzt wird.
Schwerlösliche, nahezu neutral reagierende Carbonate wie beispielsweise Calciumcarbonat( siehe PL-PS 147 078) können als säurebindene Mittel eingesetzt werden. Aber in diesem Fall setzt die CO₂ - Entwicklung und damit das Austreiben von Phosgen wesentlich zeitiger ein als beim Zulauf einer wäßrigen Alkalicarbonatlösung.
Die wäßrigen Lösungen der Alkalicarbonate oder die wäßrigen Suspensionen der Alkalihydrogencarbonate oder der Erdalkalicarbonate sind für großtechnische Phosgenierungen deshalb ungeeignet.
Für ein Äquivalent Iminostilben setzt man 0,55 - 1,00 Äqivalente Base ein - vorzugsweise jedoch nur 0,55 - 0,75 Äquivalente Base.
Beispielsweise benötigt man für 1 mol Iminostilben 0,55 - 0,75 mol NaOH, KOH oder NH₃ bzw. 0,28 - 0,38 mol CaO.
Eine weitere besondere Ausführungsform der Erfindung besteht auch darin, daß man bereits nach ca. 10-20%igem Umsatz des Iminostilbens mit dem Zulauf der wäßrigen Baselösung beginnt, wobei man die Einleitungsgeschwindigkeit des Phosgens so regelt, daß der pH-Wert der Reaktionsmischung nie in den alkalischen Bereich abgleitet.

Erwünschtenfalls kann die Reaktionsmischung zur weiteren Reinigung noch einmal mit halbkonzentrierter Salzsäure extrahiert und nach der Phasentrennung auf den pH-Wert 5 - 6 abgepuffert und danach mit Adsorptionsmitteln wie z. B. Aktivkohle und/oder Aluminiumoxid behandelt werden.

Nach Abtrennung der wäßrig sauren Phase wird die ggf. filtrierte - vorzugsweise toluenische Lösung des 5-Chlorcarbonyl-5H-dibenz/b,f/azepins (II) durch aceotrope Destillation entwässert und das Lösungsmittel abdestilliert. Die verbleibende Schmelze des 5-Chlorcarbonyl-5H-dibenz-/b,f/-azepins wird in Methanol gefällt und das kristallisierte Produkt abgetrennt und getrocknet.

### Ausführungsbeispiele

### Beispiel 1

160 g Iminostilben (I) (0,835 mol) werden in 800 ml Toluen suspendiert. Diese Suspension wird auf 35 - 40 °C erwärmt. In die gerührte Suspension leitet man Phosgen ein. Die Reaktion ist leicht exotherm. Die Temperatur der Reaktionsmischung steigt auf ungefähr 50 °C an. Bei großtechnischen Ansätzen wird die Temperatur der Reaktionsmischung durch Kühlung bei 40 - 50 °C gehalten.
Nach dem 50 %-igen Umsatz (ca. 1,5 Std. Reaktionszeit) des Iminostilbens zum 5-Chlorcarbonyl-5H-dibenz/b,f/azepin (II) steigt die Temperatur der Reaktionsmischung nicht mehr an und die Iminostilbenfarbe ist verschwunden. Unter ständigem weiteren Einleiten von Phosgen beginnt man an diesem Punkt mit dem langsamen Zulauf von verdünnter Natronlauge (20 g NaOH = 0,50 mol, gelöst in 150 ml Wasser).

Der Zulauf der Lauge ist nach 30 - 45 min beendet. Das Iminostilbenhydrochlorid geht in Lösung. Es wird weiter Phosgen eingeleitet.

Die Reaktionslösung nimmt mit fortschreitender Reaktion eine immer hellere Farbe an. Gegen Ende der Reaktion geht die Farbe des Schaumes der Reaktionslösung von gelbbraun über gelb in weiß über. Diese weiße Schaumkrone auf der gerührten Reaktionslösung und die beginnende CO₂-Entwicklung kurz vor dem Ende der Reaktion sind wichtige Hinweise auf den vollständigen Umsatz des Iminostilbens. Gegen Ende der Reaktion wird die Temperatur bei nahezu 50 °C gehalten, damit das gebildete Chlorcarbonyl-iminostilben nicht auskristallisiert. Die Phosgeneinleitung wird beendet, wenn in der Reaklösung kein Iminostilben mehr nachweisbar ist. Es werden 100 ml Wasser zugesetzt, um das gebildete NaCI in Lösung zu halten.

Anschließend heizt man die Reaktionsmischung, die den pH-Wert 1 besitzt, langsam von 50 °C auf 80 - 90 °C an. Überschüssiges Phosgen wird unter diesen Bedingungen schnell hydrolytisch zersetzt. Sobald die Reaktionsmischung phosgenfrei ist, wird die untere wäßrige, saure Phase abgetrennt und verworfen.

Diese wäßrige Phase wirkt reinigend auf das gebildete 5-Chlorcarbonyl-5H-dibenz/b,f/azepin (II), denn sie enthält mehrere aminartige Nebenprodukte (0,2 - 0,3 g) in Form ihrer Hydrochloride, vor allem 9-Methylacridin. Aus der wäßrigen Phase können die aminartigen Nebenprodukte mit NaOH freigesetzt werden.

Die toluenische Phase kann mit verdünnter Salzsäure extrahiert und mit Wasser nachgewaschen werden, wenn dies erforderlich ist. Nach erneuter Phasentrennung wird die toluenische Lösung des Chlorcarbonyl-iminostilbens (II) destilliert. Nach der Entfernung des Lösungsmittels wird die verbleibende Schmelze des Chlorcarbonyl-iminostilbens in Methanol gefällt. Die methanolische Suspension wird abgekühlt und das kristallisierte Chlorcarbonyl-iminostilben wird abgesaugt und getrocknet.

| | |
|---|---|
| Ausbeute | 197 - 200 g 5-Chlorcarbonyl-5H-dibenz/b,f/azepin (II) |
| | 93 - 94 % d. Th. |
| F | 157 - 158 °C |
| Gehalt lt. HPLC | 99,8 - 100 %-ig |
| Gehalt lt. DC | 99,7 - 99,9 %-ig |

Das auf diese Weise erhaltene 5-Chlorcarbonyl-5H-dibenz/b,f/azepin (II) liefert nach den in der Patentliteratur beschriebenen Amidierungsverfahren ein Roh-Carbamazepin, das bereits nach einer einzigen Umkristallisation aus Methanol-Wasser ein Rein-Carbamazepin ergibt, das allen Anforderungen der internationalen Pharmakopöen entspricht (maximale Einzelverunreinigung ≤ 0,01 %).

Aus der methanolischen Fällungsmutterlauge erhält man nach dem Einengen weitere 5 - 10 g 5-Chlorcarbonyl-5H-dibenz/b,f/azepin (II).

Die Gesamtausbeute beträgt somit 205 - 207 g, das sind 96,5 - 97,5 % d. Th.
Das aus der methanolischen Mutterlauge gewonnene Chlorcarbonyl-iminostilben (II) wird gesammelt und gesondert verarbeitet.

### Beispiel 2

160 g Iminostilben werden in 800 ml Toluen suspendiert. In diese Suspension wird nach Beispiel 1 bei 35 - 40 °C Phosgen eingeleitet. Nach dem 50 %-igen Umsatz des Iminostilbens beginnt man mit dem langsamen Zulauf von 70 ml 12,7 %-igem Ammoniakwasser (Dichte = 0,95 g/cm³ bei 15 °C; 0,50 mol NH₃), wobei die Phosgeneinleitung ständig fortgesetzt wird. Das verdünnte Ammoniakwasser wird in ca. 1 Std. zugetropft.

Anschließend wird die Phosgeneinleitung bis zum vollständigen Umsatz des Iminostilbens fortgesetzt. Danach wird die Reaktionslösung langsam von ca. 50 °C auf 80 - 90 °C angeheizt und bei dieser Temperatur gerührt, bis kein Phosgen mehr nachweisbar ist (Dauer der Entgiftung ca. 1 Std.).

Die entgiftete Reaktionsmischung wird mit 100 ml Wasser versetzt, um das Ammoniumchlorid zu lösen.

Danach werden 4 g Aktivkohle zugegeben. Die Mischung wird eine weitere Stunde bei 80 - 90 °C gerührt und anschließend filtriert. Die Kohle wird mit 50 ml heißem Toluen gewaschen. Vom Filtrat wird die saure wäßrige Phase abgetrennt und verworfen. Die toluenische Lösung wird nach Beispiel 1 behandelt.

| | |
|---|---|
| Ausbeute | 195 - 205 g 5-Chlorcarbonyl-5H-dibenz/b,f/azepin (II) |
| | 92,0 - 96,5 % der Theorie |
| F | 157 - 158 °C |
| Gehalt lt. HPLC | 99,8 - 100 %-ig |

Aus der methanolischen Fällbadmutterlauge erhält man nach der Destillation weitere 3- 10 g Chlorcarbonyl-iminostilben (II).

### Beispiel 3

160 g Iminostilben werden in 800 ml Chlorbenzen suspendiert. Diese Suspension wird nach Beispiel 1 mit Phosgen behandelt. Nach dem 50 %-igen Umsatz des Iminostilbens beginnt man mit dem Zulauf von verdünnter Kalilauge (30 g KOH = 0,55 mol, gelöst in 170 ml Wasser). Die Lauge wird unter weiterer Phosgeneinleitung in ungefähr 45 Minuten gleichmäßig zugetropft.

Während der Phosgenierung wird die Temperatur bei 35 - 45 °C gehalten, vorzugsweise bei 34 - 40 °C. Aufgrund der besseren Löslichkeit des 5-Chlorcarbonyl-5H-dibenz/b,f/azepins (II) in Chlorbenzen (gegenüber Toluen) kann bei ca. 40 °C gearbeitet werden, ohne daß das Reaktionsprodukt zu kristallisieren beginnt. In Toluen muß unterhalb von 40 °C mit der Kristallisation des Chlorcarbonyl-iminostilbens (II) gerechnet werden. Nach dem vollständigen Umsatz des Iminostilbens wird die Reaktionsmischung wie in Beispiel 1 oder 2 entgiftet. Um das gebildete KCI in Lösung zu halten, werden 80 ml Wasser zugesetzt.

Die Reaktionsmischung wird anschließend mit 4 g Aktivkohle versetzt, 1 Stunde bei 80 - 90 °C gerührt und danach filtriert. Die Kohle wird mit 50 ml heißem Chlorbenzen gewaschen. Die wäßrige Phase wird bei 80 - 90 °C (evtl. unter Zusatz eines oberflächenaktiven Stoffes zur Brechung der Emulsionsschicht) abgetrennt und verworfen. Die Chlorbenzenphase wird nach Beispiel 1 aufgearbeitet.

| | |
|---|---|
| Ausbeute | 190 - 193 g 5-Chlorcarbonyl-5H-dibenz/b,f/azepin (II) |
| | 90 - 91,5 %d. Th. |
| F | 157 - 158 °C |
| Gehalt lt. HPLC | 99,75 - 100 %-ig |

Aus der methanolischen Fällbadmutterlauge erhält man weitere 10 15 g Chlorcarbonyl-iminostilben (II).

### Beispiel 4

160 g Iminostilben werden in 800 ml Toluen suspendiert. Bei ca. 40 °C beginnt man mit dem Einleiten des Phosgens. Nach ca. 10 - 20 %-igem Umsatz des Iminostilbens erfolgt die langsame Zudosierung von verdünnter Natronlauge (24 g NaOH = 0,60 mol, gelöst in 200 ml Wasser).

Die Geschwindigkeit der Phosgeneinleitung und die zudosierte Menge der verdünnten Natronlauge werden so aufeinander eingeregelt, daß der pH-Wert der Reaktionsmischung während der Laugenzugabe nie in den alkalischen Bereich abgleitet. Die Phosgenierung wird bei 40 - 50°C durchgeführt. Nach dem vollständigen Umsatz des Iminostilbens wird nach Beispiel 1 aufgearbeitet.

| | |
|---|---|
| Ausbeute | 197 - 200 g 5-Chlorcarbonyl-5H-dibenz/b,f/azepin(II) |
| | 93 - 94 % der Theorie |
| F | 157 - 158 °C |

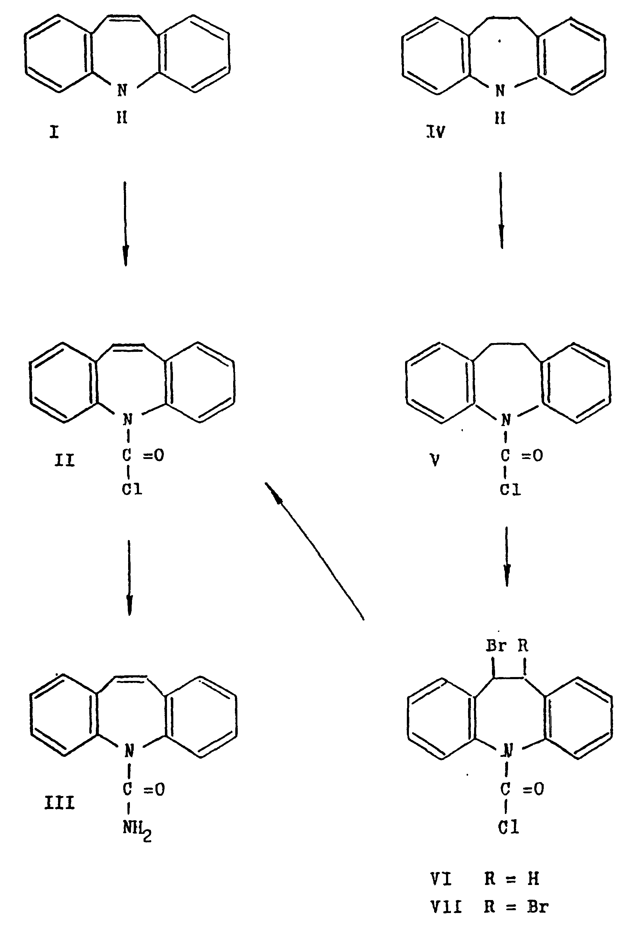

## Patentansprüche

1. Verfahren zur Herstellung von 5-Chlorcarbonyl - 5H-dibenz/b,f/azepin durch Umsetzung von Iminostilben mit Phosgen in einem wasserfreien aromatischen Lösungsmittel , **dadurch gekennzeichnet, daß** man Iminostilben in einem inerten Lösungsmittel bei Temperaturen von 20°C - 60°C mit Phosgen umsetzt, aus dem neben 5-Chlorcarbonyl-5H-dibenz/b,f/azepin sich bildenden lminostilben-Hydrochlorid durch Zugabe einer wäßrigen Alkalilauge oder von Ammoniakwasser Iminostilben freisetzt und letzeres unter Beibehaltung saurer Reaktionsbedingungen vollständig phosgeniert.

## Claims

1. Process for the production of 5-chlorocarbonyl-5H-dibenzo[*b*,*f*]azepine by reacting iminostilbene with phosgene in an anhydrous aromatic solvent, **characterised in that** iminostilbene is reacted with phosgene in an inert solvent at temperatures of 20°C-60°C, iminostilbene is liberated from the iminostilbene hydrochloride which is formed in addition to 5-chlorocarbonyl-5H-dibenzo[*b*,*f*]azepine by addition of an aqueous alkali solution or ammonia water and said iminostilbene is completely phosgenated while maintaining acidic reaction conditions.

## Revendications

1. Procédé pour la préparation de 5-chlorocarbonyl-5H-dibenz(b, f)azépine par conversion d'iminostilbène avec du phosgène dans un solvant aromatique anhydre, **caractérisé en ce que** l'on met en réaction l'iminostilbène dans un solvant inerte à des températures de 20-60°C avec du phosgène, à partir duquel en plus du 5-chlorocarbonyl-5H-dibenz(b,f)azépine se libère le chlorhydrate d'iminostilbène en formation par addition d'une lessive alcaline ou d'eau ammoniacale et le chlorhydrate d'iminostilbène se phosgénise complètement en maintenant des conditions de réaction acides.
